# FASCICULE DE BREVET EUROPEEN

(11) **EP 3 756 004 B1**
(45) Date de publication et mention de la délivrance du brevet: **09.03.2022**
(21) Numéro de dépôt: 19710058.9
(22) Date de dépôt: 15.02.2019
(51) Int. Cl.: G01N 30/76, G01N 30/30, G01N 29/02, G01N 30/62, G01N 29/036

(54) **PROCEDE D'ANALYSE D'HYDROCARBURES**
VERFAHREN ZUR ANALYSE VON KOHLENWASSERSTOFFEN
METHOD FOR ANALYSING HYDROCARBONS

(30) Priorité: 19.02.2018 FR 1851416
(43) Date de publication de la demande: 30.12.2020
(73) Titulaire: APIX Analytics, 38000 Grenoble (FR); TOTAL SE, 92400 Courbevoie (FR)
(72) Inventeur: COLINET, Eric, 38330 SAINT ISMIER (FR); ANDREUCCI, Philippe, 38500 COUBLEVIE (FR); PUGET, Pierre, 38330 SAINT-ISMIER (FR); LORIAU, Matthieu, 64140 LONS (FR); ORDONEZ VARELA, John Richard, 64230 LESCAR (FR); HAESELER, Frank, 64320 IDRON (FR)
(74) Mandataire: Regimbeau
(86) Numéro de dépôt international: PCT/FR2019/050354
(87) Numéro de publication internationale: WO 2019/158879

(56) Documents cités:
- EP-A1- 3 185 011
- US-A1- 2011 011 157
- US-A1- 2015 020 575
- MO LI ET AL: "Nanoelectromechanical Resonator Arrays for Ultrafast, Gas-Phase Chromatographic Chemical Analysis", NANO LETTERS, vol. 10, no. 10, 13 octobre 2010 (2010-10-13), pages 3899-3903, XP055086366, ISSN: 1530-6984, DOI: 10.1021/nl101586s
- J. ARCAMONE ET AL: "VLSI silicon multi-gas analyzer coupling gas chromatography and NEMS detectors", 2011 INTERNATIONAL ELECTRON DEVICES MEETING, vol. 1,17, 16 janvier 2012 (2012-01-16), pages 29.3.1-29.3.4, XP055306686, DOI: 10.1109/IEDM.2011.6131637 ISBN: 978-1-4577-0504-5

## Description

### DOMAINE DE L'INVENTION

La présente invention concerne un procédé d'analyse d'hydrocarbures se présentant sous forme liquide ou gazeuse.

### ETAT DE LA TECHNIQUE

Une chaîne de mesure pour une analyse en chromatographie en phase gazeuse d'hydrocarbures comprend un injecteur (type vanne à gaz ou injecteur liquide), une colonne de séparation, et au moins un détecteur.

Pour les besoins de l'analyse, il est nécessaire de contrôler à tout endroit la température de la chaîne de mesure.

En particulier, une règle de dimensionnement couramment admise est de maintenir la température de l'injecteur et du détecteur à une valeur constante fixée à environ 50°C au-dessus du point d'ébullition de l'échantillon gazeux à analyser (le point d'ébullition étant la température à partir de laquelle l'échantillon se trouve à l'état gazeux).

En ce qui concerne la colonne, son contrôle en température est plus subtil. En effet, la température de la colonne doit être ajustée de façon à assurer une bonne séparation des différents pics de gaz constituant l'échantillon à analyser tout en privilégiant une bonne vitesse d'analyse. Il est préférable de travailler à une température de colonne comprise entre le point de rosée et le point d'ébullition de l'échantillon.

Ainsi, il est très souvent nécessaire de maintenir la colonne dans une gamme importante de température comprise entre 20°C et 350°C.

Deux types d'analyses sont couramment mises en oeuvre. La première contrôle la température de la colonne à une valeur constante. Cette analyse isotherme est particulièrement bien adaptée pour les échantillons gazeux simples à pression atmosphérique (faible écart des points d'ébullition des analytes constituants l'échantillon) où le cycle d'analyse (intervalle de temps entre 2 analyses successives) doit être réduit à son strict minimum. La seconde approche par « programmation de température » fait appel à des rampes linéaires de température consistant à élever progressivement et par paliers la température de la colonne. On utilise cette seconde approche pour des échantillons complexes présentant un écart important des différents points d'ébullition : on citera par exemple les mélanges complexes qui se présentent à l'état liquide à température ambiante. Les rampes de températures (vitesse d'élévation en température exprimée en °C/min) sont ajustées selon les besoins en capacité de séparation ou en temps d'analyse. Ces rampes permettent également de contrôler les phénomènes d'absorption/désorption entre la phase stationnaire (substance chimique de fonctionnalisation de la colonne) et la phase mobile (gaz) dans la colonne. C'est ce partage entre la phase stationnaire et la phase mobile qui fixe la vitesse de transit d'un analyte donné et permet ainsi de séparer deux analytes différents présents dans l'échantillon à analyser.

Le détecteur situé à la sortie de la colonne permet de détecter les différentes molécules et de les convertir en pics chromatographiques ainsi séparés.

Il existe différentes technologies de détecteurs.

Parmi les détecteurs traditionnellement employés, on note les catharomètres (TCD, acronyme du terme anglo-saxon « Thermal Conductivity Detector »). Un avantage d'un tel capteur est qu'il est universel, c'est-à-dire capable de détecter tout type de gaz. Un autre avantage est que ce capteur ne détruit pas les composés gazeux de l'échantillon ; il peut donc être couplé en série avec un autre type de détecteur. Enfin, ce détecteur est compatible avec des gaz vecteurs inertes (hélium, argon, azote), même si sa sensibilité est liée au gaz vecteur utilisé. Un inconvénient de ce détecteur est que sa sensibilité est de l'ordre du ppm pour les composés légers (chaînes carbonées avec moins de 7 atomes de carbone) et d'une dizaine de ppm pour les composés plus lourds (chaînes carbonées avec plus de 7 atomes de carbone) pour les meilleurs détecteurs disponibles à ce jour sur le marché.

Un autre type de capteur est dénommé FID (acronyme du terme anglo-saxon « Flame Ionisation Detector »). Un tel capteur présente une bonne sensibilité (inférieure au ppm) vis-à-vis des hydrocarbures ; par ailleurs, cette sensibilité croît linéairement avec le nombre d'atomes de carbone. Cependant, ce détecteur est sensible uniquement aux produits carbonés (alcanes, alcènes, aromatiques) et n'est donc pas universel. Par ailleurs, son fonctionnement nécessite une flamme à l'hydrogène qui implique une consommation importante d'H₂ et qui le rend peu compatible avec les environnements explosifs. Enfin, ce détecteur présente l'inconvénient de bruler les composés gazeux formant l'échantillon.

Il existe d'autres types de détecteurs qui sont utilisés de façon plus confidentielle pour des besoins spécifiques. Parmi ceux-ci, on citera :
- les PFPD (acronyme du terme anglo-saxon « Pulse Flame Photonic Detector ») qui présentent une grande sensibilité aux produits soufrés ou phosphorés mais qui ne sont pas universels, impliquent une utilisation d'hydrogène et détruisent l'échantillon ;
- les détecteurs HID (acronyme du terme anglo-saxon « Hélium lonization Detector ») qui sont des détecteurs universels avec une limite de détection de l'ordre du ppm et qui sont sensibles à la masse de l'analyte, mais qui nécessitent une source radioactive et qui implique une grande consommation d'hélium ; un autre inconvénient de ces détecteurs est qu'ils détruisent une partie de l'échantillon ;
- les détecteurs de type nano-système électromécanique (NEMS, acronyme du terme anglo-saxon « Nano-Electro-Mechanical-System ») permettent des mesures de masse basées sur une variation de la fréquence de résonance d'un résonateur sous l'effet de l'adsorption ou de la désorption d'un analyte sur une couche fonctionnelle déposée sur le résonateur. Ces détecteurs possèdent une grande sensibilité (inférieure au ppm) sur une large gamme de molécules de C1 à C40 (non limitée aux chaînes carbonées). Etant non destructifs de l'échantillon, ils peuvent être couplés en série avec un autre détecteur. Enfin, ces détecteurs sont compatibles avec des gaz vecteurs inertes (hélium, argon, azote) sans impact notable sur la sensibilité et avec l'hydrogène (contrairement aux détecteurs HID). En revanche, ils sont peu sensibles aux composés légers (C1-C6).

L'article de Mo Li et al, Nanoelectromechanical Resonator Arrays for Ultrafast, GasPhase Chromatographic Chemical Analysis, Nano Letters, Vol. 10, no. 10, 13 octobre 2010, pages 3899-3903 décrit l'utilisation de nano-systèmes électromécaniques pour une analyse par chromatographie en phase gazeuse.

Pour l'ensemble de ces détecteurs à l'exception du NEMS, la température de fonctionnement doit être contrôlée au-dessus du point d'ébullition de l'échantillon gazeux à analyser afin d'assurer le bon transport de l'échantillon jusqu'à la partie sensible du détecteur et le bon fonctionnement du détecteur.

Pour les détecteurs NEMS, la température doit être contrôlée finement pour optimiser leur limite de détection. En effet, dans la mesure où les phénomènes d'adsorption sont minimisés avec l'augmentation de la température, on cherche généralement à maintenir le détecteur à une température suffisamment basse. Cependant, si l'on abaisse trop la température du détecteur, la réaction d'adsorption est favorisée à l'excès, pouvant entraîner une condensation de l'analyte sur le résonateur.

### EXPOSE DE L'INVENTION

Un but de l'invention est de concevoir un procédé qui puisse être mis en œuvre dans un environnement dangereux et présentant une bonne sensibilité pour une large gamme d'hydrocarbures.

A cet effet, l'invention propose un procédé d'analyse d'hydrocarbure, comprenant :
- la mise en œuvre d'une séparation chromatographique en phase gazeuse selon un premier profil de température contrôlé, pour séparer un échantillon contenant ledit hydrocarbure en une pluralité d'analytes ;
- la détection d'au moins un desdits analytes par mesure d'une variation de la fréquence de résonance d'au moins un résonateur de type nano-système électromécanique (NEMS) recouvert d'une couche fonctionnelle entraîné en vibration à sa fréquence de résonance, sous l'effet d'une adsorption ou désorption de l'analyte par la couche fonctionnelle,
   ledit procédé étant caractérisé en ce que le résonateur est soumis à un second profil de température contrôlé, inférieur au premier profil, chaque profil de température étant appliqué par des enceintes à température régulée respectives découplées thermiquement l'une de l'autre.

Par « second profil inférieur au premier profil », on entend qu'à chaque instant la température appliquée au résonateur est inférieure à la température appliquée à la colonne de chromatographie au sein de laquelle la séparation est effectuée.

De manière particulièrement avantageuse, l'écart de température entre le premier profil et le second profil est compris entre 5 et 150°C, de préférence entre 30 et 100°C, de manière encore préférée entre 40 et 60°C.

Selon une forme d'exécution préférée, la température du premier profil évolue entre 50 et 400°C. La température du second profil évolue quant à elle entre 0 et 350°C.

Selon un mode de réalisation, la détection d'au moins un desdits analytes comprend en outre la mesure d'une variation de l'amplitude de résonance du résonateur.

De manière particulièrement avantageuse, l'échantillon comprend des chaînes carbonées présentant entre 16 et 40 atomes de carbone.

Pour la mise en oeuvre dudit procédé, le résonateur est avantageusement encapsulé dans une enceinte à température régulée, ladite enceinte comprenant une unité de régulation de la température configurée pour faire varier la température à l'intérieur de l'enceinte selon le profil de température prédéfini.

Par ailleurs, la colonne de chromatographie peut être encapsulée dans une enceinte découplée thermiquement de l'enceinte dans laquelle est encapsulé le résonateur.

Selon un mode de réalisation, le procédé comprend en outre une analyse dudit au moins un analyte par un catharomètre agencé dans un même conduit fluidique que le résonateur.

De manière particulièrement avantageuse, le procédé peut comprendre la mise en oeuvre d'un traitement pour soustraire de la ligne de base de la réponse du résonateur la ligne de base d'une réponse du résonateur, dite réponse à blanc, préalablement mesurée en l'absence de circulation d'un fluide pour un même profil de température.

De manière alternative, le procédé peut comprendre la mise en oeuvre d'une mesure de la variation de la fréquence de résonance d'au moins un second résonateur, dit résonateur de référence, soumis au même profil de température mais non exposé à l'analyte, et la mise en oeuvre d'un traitement configuré pour soustraire du signal de réponse du résonateur exposé à l'analyte le signal de réponse du résonateur de référence.

De manière particulièrement avantageuse, on déduit de la mesure de la variation de la fréquence de résonance du résonateur la composition moléculaire de l'analyte.

Par ailleurs, lorsque la variation de l'amplitude de résonance du résonateur a été mesurée, on déduit de ladite mesure une caractéristique fluidique de l'analyte.

Une application de l'invention est la comparaison de deux coupes pétrolières, en mettant en oeuvre le procédé décrit plus haut pour analyser la composition de chacune desdites coupes.

Une autre application de l'invention est le dosage d'un composé déterminé dans un hydrocarbure, dans lequel on met en oeuvre le procédé décrit ci-dessus pour détecter ledit composé au sein d'un échantillon d'hydrocarbure.

Une autre application de l'invention est le dosage d'un hydrocarbure déterminé dans de l'eau, dans lequel on met en oeuvre le procédé décrit plus haut pour détecter ledit hydrocarbure au sein d'un échantillon d'eau.

L'invention concerne également un outil de forage ou un véhicule autonome pour l'exploitation d'hydrocarbures et/ou l'exploration sous-marine, comprenant un système d'analyse pour la mise en oeuvre du procédé décrit plus haut, ledit système comprenant :
- une colonne de chromatographie en phase gazeuse, agencée dans une première enceinte à température contrôlée,
- un résonateur de type nano-système électromécanique agencé dans un conduit fluidique en sortie de la colonne de chromatographie, ledit résonateur comprenant une couche fonctionnelle et étant agencé dans une seconde enceinte à température contrôlée,
- un dispositif de lecture adapté pour entraîner le résonateur en vibration à sa fréquence de résonance et pour mesurer une variation de ladite fréquence de résonance sous l'effet de l'adsorption ou de la désorption de l'analyte par la couche fonctionnelle,
   la première et la seconde enceinte étant découplées thermiquement l'une de l'autre, chaque enceinte comprenant une unité de régulation de la température, lesdites unités de régulation étant configurées pour faire varier la température dans leur enceinte respective selon des profils différents, de sorte qu'à chaque instant la température appliquée au résonateur soit inférieure à la température appliquée à la colonne de chromatographie.

De manière particulièrement avantageuse, le système d'analyse comprend en outre un catharomètre agencé dans le conduit fluidique, en amont ou en aval du résonateur.

### BREVE DESCRIPTION DES FIGURES

D'autres avantages et caractéristiques de l'invention ressortiront de la description détaillée qui va suivre, en référence aux dessins annexés sur lesquels :
- la figure 1 est une image en microscopie électronique à balayage d'un résonateur selon un mode de réalisation de l'invention, ainsi qu'une vue en coupe dudit résonateur ;
- la figure 2 est un chromatogramme brut (réponse du détecteur en fonction du temps (en secondes)) obtenu avec un détecteur selon un mode de réalisation ;
- la figure 3 est un chromatogramme (réponse du détecteur en fonction du temps (en secondes)) obtenu après soustraction du chromatogramme de la figure 2 de la ligne de base de la réponse à blanc dudit détecteur ;
- la figure 4 est un schéma de principe d'un dispositif de lecture différentielle permettant de s'affranchir des variations de ligne de base liées à des phénomènes exogènes ;
- la figure 5 présente trois chromatogrammes obtenus pour un mélange d'essence et de C28H58 avec une colonne de chromatographie encapsulée dans une enceinte distincte de celle du résonateur NEMS, pour différents écarts de température entre la colonne et le résonateur, montrant l'effet du découplage thermique entre les deux enceintes ;
- la figure 6 présente des chromatogrammes obtenus respectivement avec un détecteur FID et un détecteur NEMS pour le mélange d'essence et de C28H58 utilisé pour la réalisation de la figure 5, en appliquant l'écart de température le plus favorable entre la colonne de chromatographie et le résonateur NEMS ;
- la figure 7 illustre des chromatogrammes obtenus respectivement avec un détecteur FID et un détecteur NEMS pour un condensat, la température du résonateur NEMS suivant un profil linéaire inférieur de 60° au profil de température de la colonne de chromatographie ;
- les figures 8-12 sont des vues éclatées en perspective d'un détecteur selon un mode de réalisation de l'invention ;
- les figures 13 et 14 sont des vues en coupe partielle d'une enceinte encapsulant un résonateur selon un mode de réalisation de l'invention ;
- la figure 15 est une vue en coupe d'un mode de réalisation du conduit fluidique dans lequel est agencé le résonateur.

### DESCRIPTION DETAILLEE DE MODES DE REALISATION DE L'INVENTION

L'invention met en oeuvre un détecteur basé sur au moins un résonateur NEMS. Ce détecteur est destiné à être agencé à la sortie d'une colonne de chromatographie en phase gazeuse afin de détecter un ou des analytes contenus dans un échantillon et préalablement séparés par la colonne. L'échantillon peut être à l'état gazeux à température ambiante ; de manière alternative, il peut être liquide à température ambiante mais porté à une température supérieure à son point d'ébullition afin d'être injecté dans la colonne à l'état de vapeur, la colonne et le résonateur ainsi que le circuit fluidique qui les relie étant par ailleurs maintenus à une température supérieure à ce point d'ébullition afin d'éviter toute condensation de l'échantillon. L'échantillon est transporté dans la colonne de chromatographie et le détecteur par un gaz vecteur.

Le résonateur se présente sous la forme d'une poutre dont au moins une surface principale est recouverte d'une couche fonctionnelle qui présente une affinité chimique avec les analytes d'intérêt.

Selon les applications visées, la couche fonctionnelle peut être polaire ou apolaire.

Eventuellement, le détecteur peut comprendre plusieurs résonateurs, comprenant la même couche fonctionnelle ou une couche fonctionnelle différente, choisie en fonction des analytes d'intérêt.

La poutre est suspendue par rapport à un substrat. Selon un mode de réalisation, une extrémité de la poutre est encastrée dans le substrat et l'extrémité opposée est libre, mais d'autres solutions pour suspendre la poutre sont envisageables, par exemple un encastrement aux deux extrémités de la poutre.

A titre purement indicatif, les dimensions de la poutre d'un tel résonateur sont de l'ordre de quelques micromètres en longueur, de quelques centaines de nanomètres en largeur, et d'une centaine de nanomètres d'épaisseur.

Ainsi, selon un mode de réalisation donné à titre d'exemple, la poutre présente une longueur de 1 à 100 µm, une largeur de 50 à 500 nm voire de quelques µm et une épaisseur de 50 à 500 nm.

Le résonateur est asservi à un dispositif électronique de lecture configuré pour entraîner le résonateur en vibration à sa fréquence de résonance et pour mesurer une variation de ladite fréquence de résonance sous l'effet de l'adsorption ou de la désorption d'un analyte par la couche fonctionnelle.

Lorsqu'un analyte d'intérêt est adsorbé sur la couche fonctionnelle (ou désorbé), la masse effective du résonateur est modifiée, ce qui provoque une variation de la fréquence de résonance du résonateur. Ainsi, la mesure de la variation de la fréquence de résonance par le système de lecture permet de mesurer les variations de masse du résonateur et d'en déduire la concentration du gaz ou de la vapeur à analyser. Pour une concentration d'analyte donnée, la masse de gaz adsorbée et par conséquent la sensibilité de la mesure dépend de la constante d'équilibre entre la phase vapeur et la phase adsorbée de l'analyte. Cette constante d'équilibre dépend de la température de la surface et des caractéristiques physico-chimiques de la surface sensible. La couche fonctionnelle présente donc avantageusement une constante d'équilibre phase adsorbée / phase vapeur élevée à une température d'utilisation donnée.

Pour une concentration et une nature de la couche fonctionnelle données, une diminution de la température du détecteur augmente la masse d'analyte adsorbé, ce qui augmente le signal délivré par le détecteur. Cependant, si la température du détecteur est trop basse, la réaction d'adsorption est favorisée à l'excès, pouvant conduire de façon ultime à une condensation sur la surface du détecteur. Il existe donc une température optimale de fonctionnement du détecteur pour un analyte d'intérêt donné. Cette température optimale de fonctionnement croît approximativement comme la température d'ébullition de l'analyte.

La fabrication d'un résonateur NEMS est connue en elle-même et ne nécessite donc pas d'être décrite en détail dans le présent texte. On pourra notamment se référer aux documents [Mile2010], EP 2 008 965, WO 2012/034990 et WO 2012/034951, qui décrivent des résonateurs NEMS susceptibles d'être mis en oeuvre dans un détecteur selon l'invention.

On notera que, au lieu d'un unique résonateur NEMS, le détecteur peut comprendre un ou plusieurs réseaux de résonateurs NEMS.

Les avantages d'un réseau de résonateurs par rapport à un résonateur individuel sont multiples. D'une part, le réseau de résonateurs offre une surface totale pour la capture des espèces à analyser qui est d'autant plus grande que le nombre de poutres est élevé. Ceci permet de détecter plus finement des espèces contenues en faible concentration dans l'échantillon à analyser. Par ailleurs, l'utilisation d'un réseau de résonateurs permet de minimiser l'impact de la défaillance de l'un d'entre eux, qui est compensée par le fonctionnement des autres résonateurs du réseau, améliorant ainsi la robustesse du détecteur. Enfin, pour un réseau de N résonateurs NEMS, on devrait atteindre, en théorie, un gain en limite de détection de l'ordre de √N en termes de signal (ou de l'ordre de N en termes de puissance).

On pourra se reporter au document WO 2014/053575 pour la description d'un réseau de résonateurs NEMS pouvant être mis en oeuvre dans un détecteur selon la présente invention.

Par ailleurs, dans le cas où plusieurs réseaux de résonateurs NEMS sont employés au sein du détecteur, il est possible de fonctionnaliser ces réseaux avec une couche fonctionnelle différente d'un réseau à l'autre.

Bien que l'on utilise dans la suite du texte le terme « résonateur NEMS » au singulier, il est entendu que la description s'applique également à une pluralité de résonateurs NEMS, agencés ou non en réseau.

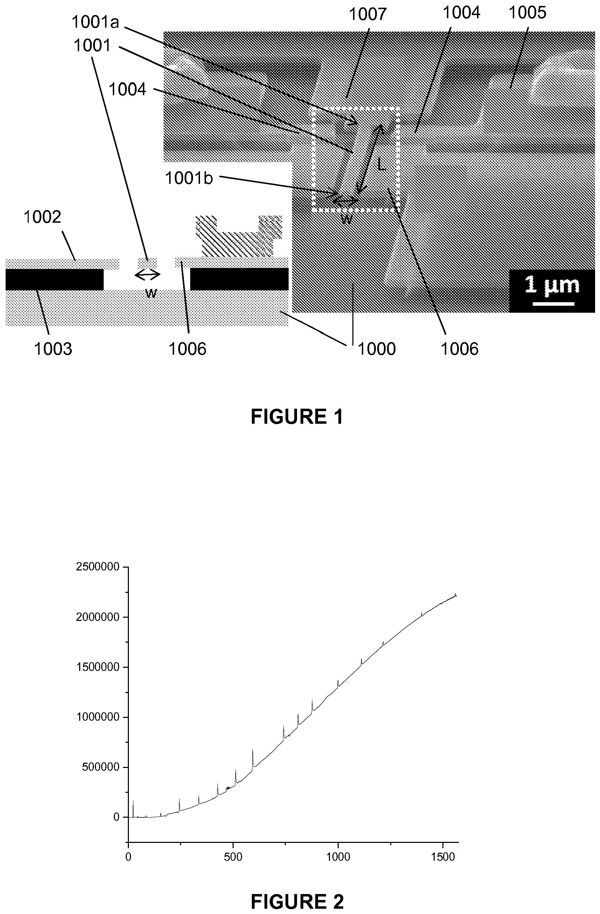

La figure 1 est une vue au microscope électronique à balayage d'un résonateur NEMS susceptible d'être mis en oeuvre dans un détecteur selon l'invention, ainsi qu'une vue schématique en coupe transversale.

Ledit résonateur est avantageusement formé sur un substrat 1000 semi-conducteur, par exemple de silicium. Le substrat 1000 est avantageusement recouvert d'une couche 1003 électriquement isolante (par exemple, en oxyde de silicium) et d'une couche 1002 de silicium, pour former un substrat de type silicium sur isolant (SOI, acronyme du terme anglo-saxon « Silicon On Insulator »).

Le résonateur comprend une poutre 1001 de longueur L et de largeur w.

La poutre 1 est suspendue par rapport au substrat support 2, à l'exception d'un encastrement de l'une de ses extrémités 1001a dans une partie du substrat 1000, en saillie par rapport au plan du substrat qui s'étend sous la poutre.

L'autre extrémité 1001b de la poutre est quant à elle libre.

De manière connue en elle-même, une telle poutre peut être formée dans la couche 1002, au moyen d'une gravure permettant de délimiter la poutre et d'éliminer la partie de la couche électriquement isolante 1003 située sous la poutre 1001, afin de libérer celle-ci.

De part et d'autre de la poutre s'étendent deux jauges de contrainte 1004, par exemple piézorésistives, qui sont également suspendues par rapport au substrat 1000.

Avantageusement, lesdites jauges sont, comme la poutre, gravées dans le substrat SOI et présentent au moins un plan en commun avec la poutre.

Ces jauges sont avantageusement en matériau semi-conducteur dopé, présentant de préférence une concentration en dopants supérieure à 10¹⁹ atomes/cm³.

De préférence, ledit matériau semi-conducteur dopé est du silicium dopé.

L'intersection entre chacune des jauges et la poutre se trouve à une distance de la région d'encastrement de la poutre, choisie pour maximiser la contrainte exercée sur la jauge lors de la déflexion de la poutre.

Chacune des jauges 1004 est reliée à une électrode 1005, lesdites électrodes permettant l'application respectivement de potentiels constants de signes opposés.

Dans d'autres modes de réalisation du résonateur, il est possible de n'employer qu'une jauge de contrainte en matériau semi-conducteur dopé.

Le résonateur comprend en outre un dispositif d'actionnement électrostatique de la poutre 1001 qui, comme représenté ici, peut comprendre deux électrodes 1006 s'étendant dans le même plan que la poutre et disposées de part et d'autre de celle-ci, à une distance déterminée.

Les électrodes 1006 sont destinées à recevoir respectivement un signal électrique d'excitation et un signal de signe opposé, et constituent donc deux entrées du résonateur.

Sous l'application d'un signal électrique présentant une fréquence correspondant à la fréquence de résonance à vide de la poutre, la poutre est entraînée en vibration dans un plan parallèle au substrat.

Par fréquence de résonance à vide de la poutre, on entend la fréquence de résonance de la poutre en l'absence de l'échantillon à analyser.

Selon une forme d'exécution, la mesure de la variation de résistance électrique des jauges piézorésistives est effectuée entre l'extrémité encastrée de la poutre et la jonction entre la poutre et les jauges.

Le signal de sortie du résonateur est ainsi fourni à une électrode de connexion 1007 située au niveau de l'extrémité encastrée de la poutre, en vue de la lecture dudit signal.

Cette méthode de mesure n'est cependant pas exclusive et le signal de sortie peut être fourni par d'autres moyens ; par exemple, il est possible d'appliquer une tension de polarisation au niveau de l'électrode et de mesurer la tension aux bornes de l'ensemble des deux jauges pour en déduire la variation de leur résistance électrique

L'homme du métier pourra donc ajuster la conception de la polarisation de la(les) jauge(s) de contrainte et de la mesure de leur réponse sans pour autant sortir du cadre de la présente invention. Par ailleurs, on pourra utiliser un autre mode d'actionnement sans sortir du champ de l'invention.

De manière particulièrement avantageuse, le résonateur NEMS peut être formé sur une puce de quelques millimètres de côté, ladite puce pouvant être embarquée sur un circuit imprimé comme cela sera décrit en détail plus bas.

Pour permettre de contrôler la température du résonateur NEMS, celui-ci est encapsulé dans une enceinte à température régulée.

Le volume de l'enceinte est choisi pour être juste suffisant pour englober le résonateur et la puce sur laquelle il est formé, en minimisant les espaces vides afin d'optimiser la consommation d'énergie nécessaire pour réguler la température dans l'enceinte.

La puce sur laquelle est formé le NEMS étant de faibles dimensions, le volume intérieur de l'enceinte peut être particulièrement faible (de l'ordre de quelques mm³ ou dizaines de mm³). Ceci présente plusieurs avantages. D'une part, la puissance électrique à mettre en oeuvre pour réguler la température au sein de l'enceinte est réduite. D'autre part, pour une puissance donnée, la vitesse de chauffe et de refroidissement est augmentée par rapport à une enceinte de volume plus grand. Enfin, le découplage thermique de l'enceinte du détecteur vis-à-vis de la colonne de chromatographie est également plus aisé.

Le contrôle de la température dans l'enceinte est assuré par une unité de régulation de la température.

Ladite unité de régulation peut comprendre des moyens de chauffage et/ou des moyens de refroidissement, ainsi qu'un capteur de température et une boucle d'asservissement permettant d'appliquer à l'intérieur de l'enceinte une température conforme à un profil déterminé. Ledit profil de température peut être défini par un utilisateur en fonction de la composition de l'échantillon à analyser et des analytes recherchés.

La boucle d'asservissement comprend typiquement un calculateur communiquant avec une interface utilisateur et configuré pour recevoir le profil de température à appliquer et des données de mesure du capteur de température, et pour, à partir de ces éléments d'entrée, commander les moyens de chauffage et/ou de refroidissement pour atteindre la température souhaitée au sein de l'enceinte au cours du temps.

Le capteur de température peut être un thermomètre à résistance de platine couramment utilisé dans les dispositifs électroniques, notamment du type Pt100.

De manière particulièrement avantageuse, l'unité de régulation comprend un élément chauffant agencé à l'intérieur de l'enceinte. Pour des raisons de compacité, ledit élément chauffant peut être une résistance chauffante alimentée par un courant électrique.

De préférence, l'unité de régulation comprend en outre un système de refroidissement. Différentes technologies de refroidissement sont envisageables : ventilateur, cellule Peltier, circuit fluidique de refroidissement, éventuellement combinées. L'homme du métier est en mesure de choisir et de dimensionner le système de refroidissement en fonction de l'agencement de l'enceinte et des performances attendues. Le système de refroidissement permet en particulier de refroidir rapidement le volume intérieur de l'enceinte après une phase de détection à température élevée, et autorise ainsi la mise en oeuvre rapide d'une nouvelle phase de détection à température plus basse.

L'enceinte n'est pas nécessairement fermée hermétiquement ni isolée thermiquement de l'extérieur. Au contraire, elle peut comprendre des évents qui permettent une évacuation plus rapide de la chaleur lorsque l'on souhaite refroidir le résonateur. Par ailleurs, le volume intérieur de l'enceinte peut être au moins en partie chauffé par transfert thermique passif à partir de l'extérieur de l'enceinte (par exemple du fait de sa proximité vis-à-vis de la colonne de chromatographie qui est elle-même chauffée).

Outre la masse effective du résonateur, la fréquence de résonance du résonateur NEMS dépend également de la température du détecteur, du débit du gaz vecteur et d'autres facteurs exogènes.

De ce fait, la ligne de base du signal de mesure varie avec la température. Il y a donc une superposition entre le signal utile à la mesure (i.e. les variations de fréquence de résonance du résonateur NEMS liées à une adsorption/désorption du gaz) et un signal de fond (variation de température du résonateur NEMS, et autres facteurs exogènes faisant varier la fréquence de résonance du résonateur) non utile.

A titre d'exemple la figure 2 montre un chromatogramme brut obtenu avec un résonateur NEMS dont la température de travail varie selon un profil de température linéaire variant entre 40 et 250°C à raison de 20°C par minute. On observe bien la variation de fréquence et le faible rapport signal sur fond bruit lié à la rampe de température appliquée au NEMS, qui pénalise l'identification des différents pics.

De manière avantageuse, on met donc en oeuvre une lecture dite différentielle du résonateur NEMS qui permet de réduire ces variations indépendantes de la variation de masse créée par adsorption d'un analyte d'intérêt et faire ressortir uniquement les variations de la fréquence de résonance du résonateur NEMS par adsorption/désorption du gaz.

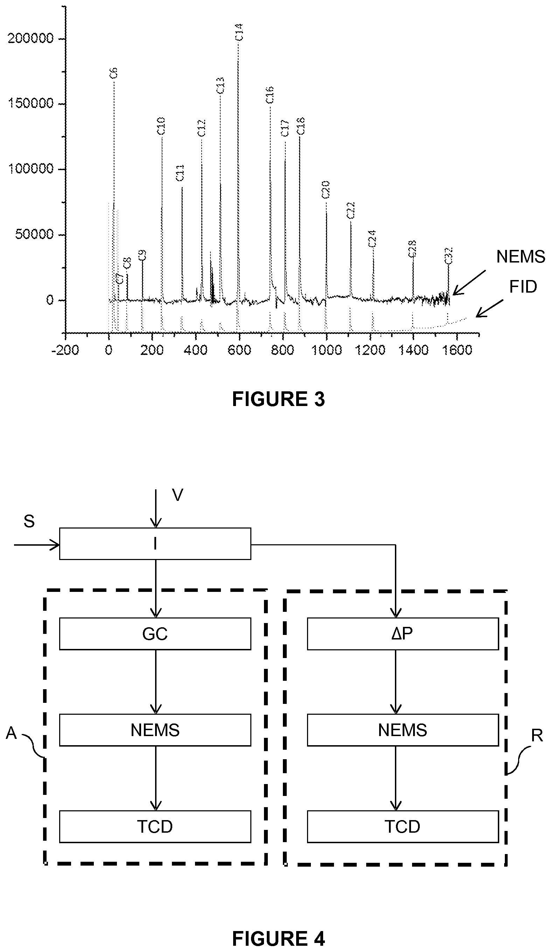

A cet effet, une première approche consiste à réaliser une analyse dite à blanc pour laquelle aucun échantillon n'est injecté dans la colonne de chromatographie tout en appliquant les profils de températures sur la colonne et le détecteur NEMS nécessaires à l'analyse. Pour cette analyse, on peut injecter dans le système uniquement le gaz vecteur, ou travailler avec un circuit de gaz vide. De cette façon, on peut recueillir les variations de la ligne de base du détecteur liées à tout autre phénomène que l'adsorption et la désorption de gaz. Ensuite, une analyse est réalisée en injectant l'échantillon et en appliquant les mêmes profils de températures qu'au cours de l'analyse à blanc. Enfin, dans un traitement dit hors ligne, on soustrait la ligne de base mesurée à blanc de la ligne de base mesurée avec l'injection de l'échantillon de manière à ne conserver que les variations de lignes de base du détecteur liées à l'adsorption/désorption des différents pics de gaz. La figure 3 (courbe NEMS) illustre le résultat d'un tel traitement réalisé sur le chromatogramme de la figure 2. A titre de comparaison, la réponse d'un détecteur FID est affichée sur le chromatogramme (courbe FID), ce qui permet de vérifier la bonne correspondance des pics détectés avec les deux techniques.

Les chromatogrammes des figures 2 et 3 ont été obtenus pour un mélange simple d'hydrocarbures. La figure 3 montre que des composés jusqu'à 32 atomes de carbone ont pu être détectés par le résonateur NEMS, ce qui confirme la capacité du résonateur NEMS à analyser des hydrocarbures.

Une autre approche de lecture différentielle consiste à mesurer simultanément au cours de la même phase d'analyse la variation de fréquence de résonance de deux résonateurs en différentiel (ou, si un détecteur TCD est associé au détecteur NEMS, deux couples formés chacun d'un résonateur NEMS et d'un détecteur TCD). Comme illustré sur la figure 4, l'un de ces couples est agencé sur la voie d'analyse A et mesure donc les différents pics d'analytes et tout autre phénomène exogène. L'autre couple est agencé sur une voie R dite de référence et ne mesure donc que les phénomènes exogènes. Le système comprend, en amont des deux voies, un injecteur I qui reçoit d'une part l'échantillon S sous forme gazeuse et vapeur et le gaz vecteur V et qui les mélange avant de les injecter, d'une part dans la colonne de chromatographie notée GC, qui est sur la voie d'analyse A, et d'autre part dans un conduit ΔP présentant une perte de charge identique à celle de la colonne, sur la voie de référence R. Au travers d'une électronique différentiant les signaux de mesure issus des deux couples de détecteurs, on ne conserve donc que les signaux issus de l'adsorption/désorption des différents pics de gaz. Cette approche est préférée dans la mesure où elle ne nécessite pas d'analyse à blanc ni de traitement hors ligne.

Les résonateurs NEMS sont capables d'avoir des interactions (dites interactions fluidiques) non négligeables avec un gaz environnant.

Par conséquent, outre la variation de fréquence de résonance du résonateur, il est possible de mesurer la variation d'amplitude de résonance due aux interactions fluidiques entre le résonateur et l'échantillon.

Comme décrit dans le document EP 2 878 942, on peut déduire de cette variation d'amplitude une caractéristique fluidique de l'échantillon. Cette caractéristique fluidique est avantageusement une viscosité, une viscosité effective, un libre parcours moyen des molécules, un débit et/ou une conductivité thermique de l'échantillon. Par « viscosité effective », on entend dans le présent texte un paramètre de viscosité tenant compte du régime de raréfaction des gaz dans l'équation de Reynolds simplifiant l'équation de Navier-Stokes (cf. paragraphe 5.1 de [Bao2007]).

Il a par ailleurs été démontré que le contraste entre les caractéristiques fluidiques du gaz vecteur et celles de l'échantillon à analyser est d'autant plus grand que la température à laquelle est soumis le résonateur NEMS est élevée. Dans le système décrit dans le document EP 2 878 942, le résonateur NEMS est chauffé par effet Joule afin de minimiser l'influence des phénomènes d'adsorption par rapport aux interactions fluidiques.

D'autre part, le fait de chauffer le résonateur NEMS permet de diminuer la viscosité effective de l'échantillon et du gaz vecteur et donc d'augmenter la variation d'amplitude mesurée par le dispositif de lecture.

La présente invention peut donc mettre à profit le fait que le résonateur soit chauffé dans l'enceinte pour mettre en oeuvre, avec une bonne sensibilité, une mesure d'au moins une caractéristique fluidique selon le principe décrit dans le document EP 2 878 942, tout en s'affranchissant du système de chauffage par effet Joule utilisé dans ce système.

La combinaison de la mesure de variation de fréquence et de la mesure de variation d'amplitude permet d'obtenir davantage d'informations sur l'échantillon, ce qui permet de différencier plus précisément des analytes présentant une réponse proche.

En raison du faible niveau de réponse du résonateur NEMS aux composés légers (C1-C6), il peut être souhaitable, selon les besoins applicatifs, de le coupler en série à un micro-catharomètre (également appelé détecteur TCD dans la suite du texte), qui est plus sensible à ces espèces. Les deux détecteurs étant non destructifs, le détecteur TCD peut être agencé dans le même conduit fluidique que le résonateur NEMS, en amont ou en aval de celui-ci.

Le détecteur TCD peut être mis en place dans la même enceinte que le détecteur NEMS, afin de faciliter l'intégration desdits détecteurs, par exemple sur une même puce ou un même circuit imprimé. De manière alternative, le détecteur TCD peut également être implémenté en dehors de l'enceinte du détecteur NEMS, sa sensibilité n'étant pas directement influencée par sa température de travail.

Le détecteur TCD est connu en lui-même et ne sera donc pas décrit plus en détail dans le présent texte.

Le détecteur qui vient d'être décrit est particulièrement adapté à la mise en oeuvre dans un système d'analyse de gaz comprenant une colonne de chromatographie en phase gazeuse et un détecteur agencé à la sortie de ladite colonne.

La colonne de chromatographie est agencée dans une enceinte régulée en température et découplée thermiquement de l'enceinte dans laquelle le résonateur est encapsulé. En amont de la colonne de chromatographie, un injecteur permet de vaporiser l'échantillon et le mélanger au gaz vecteur. Pour des hydrocarbures, on met généralement en œuvre une injection par division (« split » selon la terminologie anglo-saxonne), c'est-à-dire que l'on injecte dans la colonne seulement une fraction de l'échantillon vaporisé entraîné par le gaz vecteur.

Un conduit fluidique (par exemple sous la forme d'un tube capillaire) relie la sortie de la colonne de chromatographie à l'entrée du détecteur.

Pour maximiser le découplage thermique entre les deux enceintes, il est préférable d'augmenter la distance entre les enceintes et de chauffer le conduit de liaison pour éviter tout point froid dans lequel une condensation pourrait se produire.

Grâce à ce découplage thermique, il est possible d'ajuster indépendamment les profils de température dans chacune des deux enceintes, ce qui permet de maîtriser la température du résonateur NEMS quelle que soit la température de la colonne de chromatographie.

Contrairement aux autres détecteurs employés en chromatographie, un résonateur NEMS présente une température optimale de détection qui dépend de l'analyte d'intérêt.

Si le résonateur est à une température trop élevée, l'adsorption du gaz est moins efficace et par conséquent la sensibilité de mesure est plus basse. Par ailleurs, une température trop basse du résonateur peut engendrer une déformation des pics (qui sont, pour un système analytique bien dimensionné, de forme gaussienne) en sortie de colonne (se traduisant par une traîne du pic plus importante), diminuant le pouvoir de séparation de la colonne de chromatographie, et/ou un encrassement de la couche fonctionnelle (les sites d'adsorption n'étant pas libérés), diminuant son efficacité dans le temps.

Pour optimiser les performances du résonateur NEMS, il est nécessaire de contrôler finement sa température de travail pour la maintenir proche de cette valeur optimale pour chaque analyte d'intérêt. Il est donc utile d'adapter dynamiquement la température du résonateur en fonction des analytes qui sortent de la colonne, de même que la colonne de chromatographie est régulée en température pour contrôler la vitesse d'analyse / et la séparation. Pour couvrir un large panel de composés gazeux à détecter, il est souhaitable de contrôler le résonateur dans une gamme de température aussi grande que possible, typiquement entre la température ambiante (20°C) et 350°C.

Pour de nombreuses applications d'analyse de mélanges complexes, il est souvent opportun de réaliser un profil de montée en température, par exemple sous la forme d'une rampe linéaire, sur la colonne de chromatographie pour séparer les composés gazeux retenus par la colonne. La température de travail du résonateur NEMS ne doit pas être nécessairement identique à celle de la colonne pour optimiser ses performances de détection. Dans la plupart des cas, il est d'ailleurs préférable que la température du résonateur NEMS soit inférieure à celle de la colonne, ce que permet le découplage thermique entre les deux enceintes.

Selon un mode de réalisation, la régulation de la température du résonateur NEMS peut être basée sur la température dans l'enceinte de la colonne de chromatographie. A cet effet, l'élément chauffant de l'enceinte contenant la colonne de chromatographie comprend un capteur de température (de type Pt100 ou thermocouple), afin de mesurer la température de la colonne de chromatographie à tout moment. La température de l'enceinte contenant le résonateur NEMS peut être ajustée en temps réel en fonction de la température dans l'enceinte contenant la colonne de chromatographie. En d'autres termes, la température T_NEMS du résonateur NEMS est adaptée selon une loi T_NEMS = f (T_GC) où f est une fonction analytique programmable et T_GC la température dans l'enceinte de la colonne de chromatographie, mesurée par le capteur de température précité.

Un exemple de profil de température du résonateur NEMS consiste par exemple à appliquer un écart de température constant entre la température de la colonne et celle du résonateur NEMS. La loi de régulation de la température est alors T_NEMS = T_GC - ΔT, où ΔT est une constante.

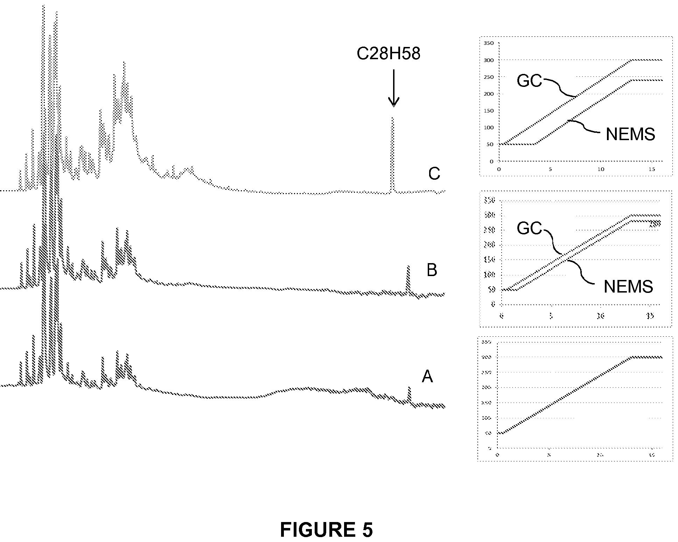

La figure 5 présente trois chromatogrammes obtenus avec une colonne de chromatographie encapsulée dans une enceinte distincte de celle du résonateur, montrant l'effet du découplage thermique entre les deux enceintes.

L'échantillon à analyser est une solution d'essence relativement légère comprenant une concentration de C28H58, qui est un hydrocarbure relativement lourd, de 5000 mg/L.

Le profil de température dans l'enceinte de la colonne de chromatographie est identique dans les trois cas : il s'agit d'une rampe linéaire de montée en température de 20°C par minute, entre 50°C et 300°C (notée GC sur les profils de température présentés à droite des courbes).

Le chromatogramme A a été réalisé en appliquant dans l'enceinte du résonateur une rampe de montée en température (notée NEMS) identique à celle de la colonne (notée GC) (écart entre les deux rampes = 0°C).

Le chromatogramme B a été réalisé en appliquant dans l'enceinte du résonateur une rampe de température de 20°C par minute, mais déclenchée avec un retard d'une minute, de sorte que l'écart entre les deux rampes de température est de 20°C.

Le chromatogramme C a été réalisé en appliquant dans l'enceinte du résonateur une rampe de température de 20°C par minute, mais déclenchée avec un retard de trois minutes, de sorte que l'écart entre les deux rampes de température est de 60°C.

La comparaison des chromatogrammes A, B et C montre que l'écart de température le plus élevé entre le profil de température de la colonne et celui du résonateur procure la meilleure sensibilité au pic de C28H58.

On notera cependant qu'un écart de température trop important ne procure plus une telle amélioration.

Par conséquent, pour la détection d'hydrocarbures présentant de longues chaînes carbonées, on choisit de préférence un écart de température entre l'enceinte du résonateur et l'enceinte de la colonne de chromatographie compris entre -5 et -150°C, de préférence entre -30 et -100°C, voire entre -30 et -70°C, et de manière encore préférée entre -40 et -60°C, par exemple égal à -50°C.

Naturellement, d'autres stratégies de profil de température du résonateur NEMS peuvent être adoptées selon les applications visées. En particulier, l'écart de température entre la colonne de chromatographie et le résonateur NEMS n'est pas nécessairement constant au cours du temps.

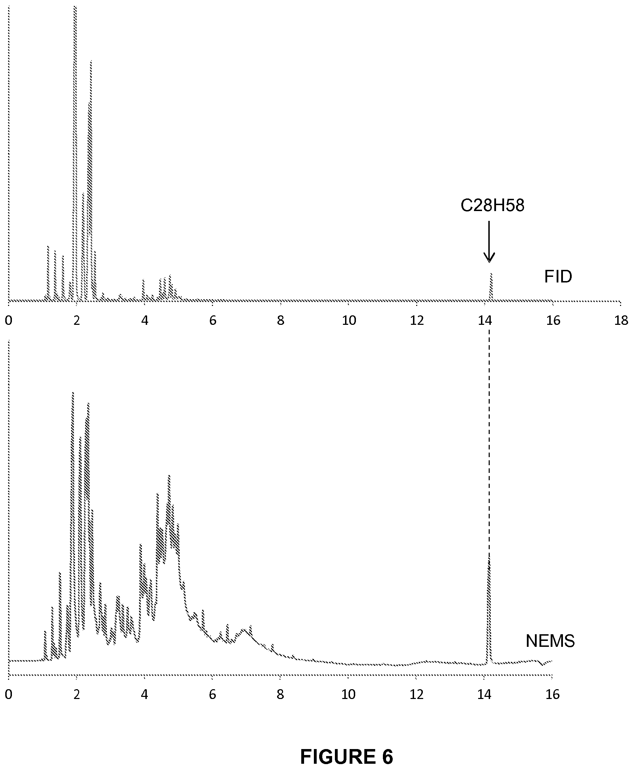

Les chromatogrammes de la figure 6 ont été obtenus pour le même mélange d'essence et de C28H58, respectivement avec un détecteur FID (courbe supérieure) et un résonateur NEMS selon l'invention, en respectant un écart de température de -60°C entre la colonne de chromatographie et le résonateur. Ces deux courbes n'ont pas la même échelle selon l'axe des ordonnées, mais permettent de vérifier que l'on détecte bien le pic de C28H58 avec le résonateur NEMS.

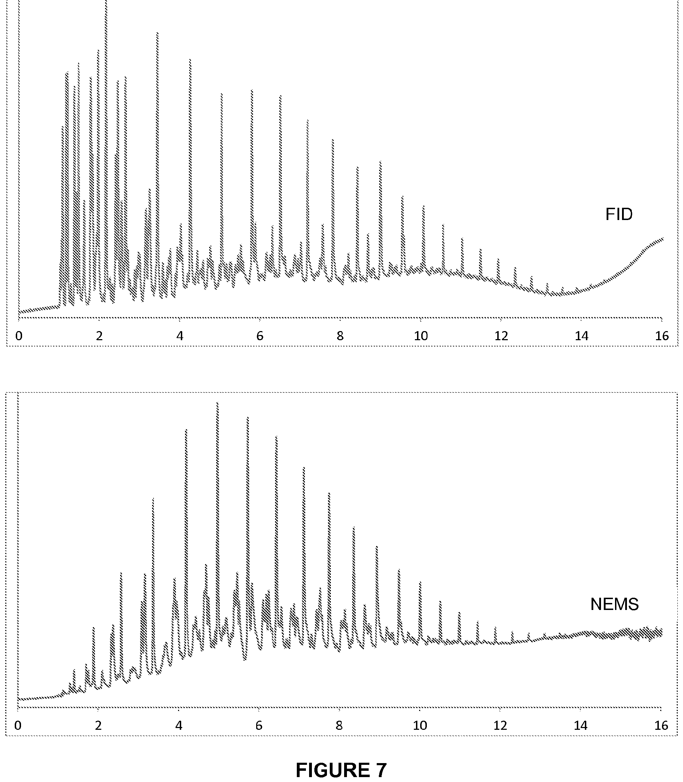

Les chromatogrammes de la figure 7 ont été quant à eux obtenus à partir d'un condensat qui est une coupe pétrolière réelle. La courbe supérieure représente le signal du détecteur FID, la courbe B représente le signal du détecteur NEMS, la différence de température entre la colonne de chromatographie et le résonateur NEMS étant de -60°C.

Bien que le condensat constitue un mélange beaucoup plus complexe que dans le cas précédent, on observe une bonne corrélation entre le signal FID et le signal NEMS, et notamment des pics du signal NEMS bien marqués.

Pour éviter la saturation du résonateur NEMS, il pourra être nécessaire de veiller à ne pas injecter un échantillon trop important dans la colonne de chromatographie. L'homme du métier pourra donc être amené à ajuster le rapport de division (« split ratio » selon la terminologie anglo-saxonne) entre le volume d'échantillon effectivement injecté dans la colonne de chromatographie et le volume total de l'échantillon vaporisé.

On notera que le fait de chauffer le résonateur NEMS, et en particulier de manière dynamique selon un profil de température, va à l'encontre de la pratique habituelle dans le domaine de l'analyse de gaz. En effet, s'agissant d'une détection gravimétrique dont la sensibilité est directement liée à un mécanisme d'adsorption, qui est pénalisée par la chaleur, il est au contraire d'usage de maintenir le résonateur NEMS à une température relativement basse et stable. D'ailleurs, la couche fonctionnelle polymère habituellement utilisée pour fonctionnaliser le résonateur NEMS n'est pas adaptée pour fonctionner à des températures aussi élevées que celles prévues dans la présente invention.

Ainsi, le système selon l'invention procure une alternative particulièrement pertinente aux détecteurs FID pour l'analyse d'hydrocarbures, notamment d'hydrocarbures lourds. En effet, le détecteur NEMS présente des performances au moins égales à celle d'un détecteur FID tout en s'affranchissant de ses défauts (caractère destructif, limitation aux chaînes carbonées, présence d'hydrogène et d'une flamme), ce qui permet une utilisation en environnement contraint.

### Application 1 : description moléculaire des hydrocarbures et dosage

Selon un mode de réalisation, la couche fonctionnelle recouvrant le résonateur NEMS est une phase apolaire. Un tel résonateur agencé à la sortie d'une colonne de chromatographie en phase gazeuse permet de décrire à l'échelle moléculaire, et de manière répétable, la composition de fluides pétroliers à l'état gazeux ou liquide, d'un point de vue qualitatif et quantitatif.

Selon un autre mode de réalisation, la couche fonctionnelle recouvrant le résonateur NEMS est une phase polaire. Un tel résonateur agencé à la sortie d'une colonne de chromatographie en phase gazeuse permet de décrire à l'échelle moléculaire, et de manière répétable, la composition en molécules soufrées, oxygénées et azotées de fluides pétroliers ou raffinés à l'état gazeux ou liquide, d'un point de vue qualitatif et quantitatif.

Dans les deux cas, l'analyse moléculaire qui est réalisée grâce au système selon l'invention permet un grand nombre d'applications dans le domaine pétrolier.

Une première application est l'analyse de la compartimentalisation et connectivité des réservoirs. Ainsi, l'analyse moléculaire permet de déterminer les similitudes et les différences dans la composition en hydrocarbures de 1 à 50 atomes de carbone par des approches statistiques afin de déterminer les zones qui peuvent être produites par un puits et de mieux comprendre les écoulements de fluides dans les réservoirs pétroliers.

Une autre application concerne l'allocation de production. A partir de l'analyse moléculaire, il est en effet possible de calculer par des approches statistiques les proportions de fluides provenant de différents réservoirs qui contribuent à la production en mélange : d'un même puits, de différents puits entre eux, de différentes plateformes entre elles et de différents champs entre eux.

Une autre application concerne l'évaluation de l'intégrité des puits. A partir de l'analyse moléculaire, il est possible de d'identifier par des approches statistiques l'origine de fluides qui apparaîtraient dans un annulaire de puits pétrolier en comparant la composition des produits trouvés dans un annulaire avec celles des fluides de tous les réservoirs traversés et/ ou processus intra annulaire.

Une autre application concerne la prédiction et la description de la composition des fluides dans une formation réservoir. La description des compostions moléculaires des fluides obtenue grâce au système d'analyse de gaz peut être utilisée dans la détermination des propriétés des fluides (en particulier leur état de phase en conditions de réservoir, leur densité, leur viscosité, etc.) via des modèles thermodynamiques.

Une autre application concerne la description de la composition fluide à une profondeur donnée au sein d'une formation par une mesure directe dans les conditions de profondeur. Cette composition peut ensuite être comparée constituant par constituant a celle d'une autre profondeur pour en déterminer l'évolution.

Enfin, dans le cas où la couche fonctionnelle est une phase polaire, l'analyse moléculaire permet d'assurer un suivi de coupes sulfurées dans les charges d'unités d'hydrodésulfuration dans les raffineries.

Les différentes techniques d'exploitation de l'analyse moléculaire fournie par le système d'analyse de gaz sont connues en elle-même et ne seront donc pas décrites en tant que telles dans le présent texte.

### Application 2 : description moléculaire des hydrocarbures et dosage dans l'eau

Selon un mode de réalisation, la couche fonctionnelle recouvrant le résonateur NEMS est une phase apolaire. Un tel résonateur agencé à la sortie d'une colonne de chromatographie en phase gazeuse couplée à un système d'extraction / préconcentration permet de décrire à l'échelle moléculaire, et de manière répétable, la composition de tout type d'hydrocarbure dans l'eau.

Selon un autre mode de réalisation, la couche fonctionnelle recouvrant le résonateur NEMS est une phase polaire. Un tel résonateur agencé à la sortie d'une colonne de chromatographie en phase gazeuse couplée à un système d'extraction / préconcentration permet de décrire à l'échelle moléculaire, et de manière répétable, la composition de tout type d'hydrocarbure soufré, oxygéné ou azoté dans l'eau.

Dans les deux cas, l'analyse moléculaire qui est réalisée grâce au système selon l'invention permet un grand nombre d'applications à des analyses environnementales dans le domaine pétrolier.

Une première application concerne le dosage des hydrocarbures aromatiques polycycliques (HAP), des polychlorobiphényles (PCB), des BTEX (benzène, toluène, éthylbenzène, xylène), des alcanes, des alcènes et/ou autres molécules cibles environnementales dans des eaux synthétiques ou réelles (de mer, de rejets, de rivières, de réservoir, etc.) après extraction liquide-liquide (solvant organique non miscible à l'eau).

Une autre application concerne le dosage des HAP, PCB, BTEX, alcanes, alcènes et/ou autres molécules cibles environnementales dans des eaux synthétiques ou réelles après extraction de l'espace de tête (analyse dite « Headspace »).

Une autre application concerne le dosage des HAP, PCB, BTEX, alcanes, alcènes et/ou autres molécules cibles environnementales dans des eaux synthétiques ou réelles après extraction en phase solide de type SPE (acronyme du terme anglo-saxon « solid-phase extraction »), SPME acronyme du terme anglo-saxon « solid-phase microextraction »), SBSE (acronyme du terme anglo-saxon « Stir bar sorptive extraction »), ou sur tout autre matériau solide ou membrane capable de créer des coefficients de partages favorables avec l'eau.

Une autre application concerne le traçage des fuites d'hydrocarbures naturelles ou industrielles dans l'environnement naturel, non seulement pour des besoins d'exploration mais également pour le contrôle des installations de fond (pipes, vannes, risers, etc ...) et le suivi de la qualité de l'eau. L'analyse moléculaire des hydrocarbures dans l'eau peut en effet apporter des informations cruciales telles que la détection précoce des fuites, l'identification de la source de fuite, etc.

### Application 3 : suivi de traceurs

Que la couche fonctionnelle recouvrant le résonateur NEMS soit une phase apolaire ou une phase polaire, le résonateur agencé à la sortie d'une colonne de chromatographie en phase gazeuse permet de détecter tout type de traceur naturel ou synthétique dans les fluides pétroliers et par conséquent de suivre ledit traceur de manière sélective.

Une application particulièrement avantageuse de l'invention est alors l'analyse qualitative et quantitative de traceurs artificiels (injectés ou intégrés dans les complétions) à des fins d'allocation de production et de détermination d'origine des fluides.

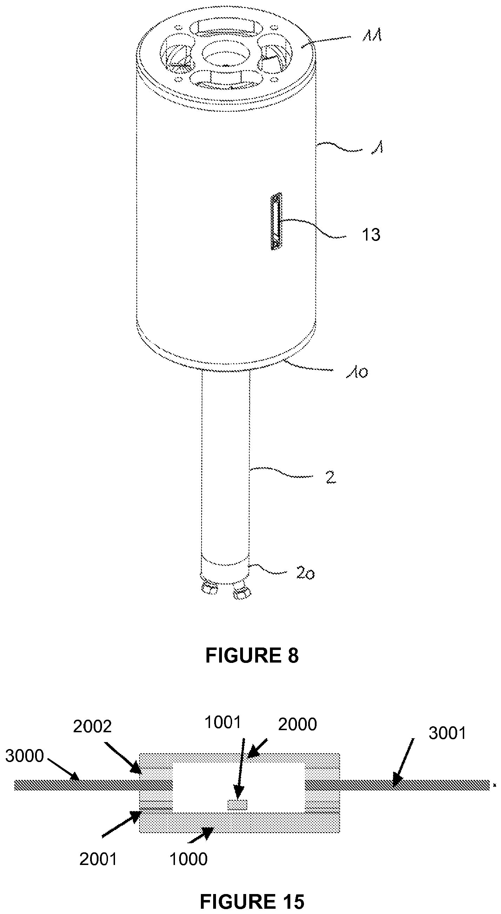

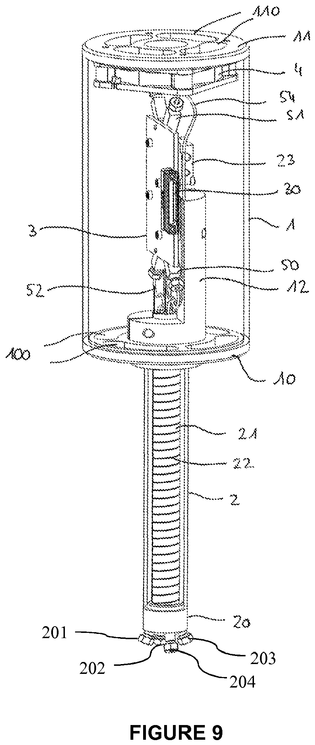

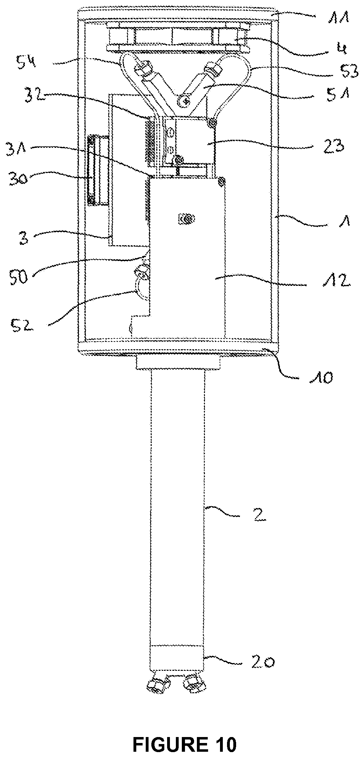

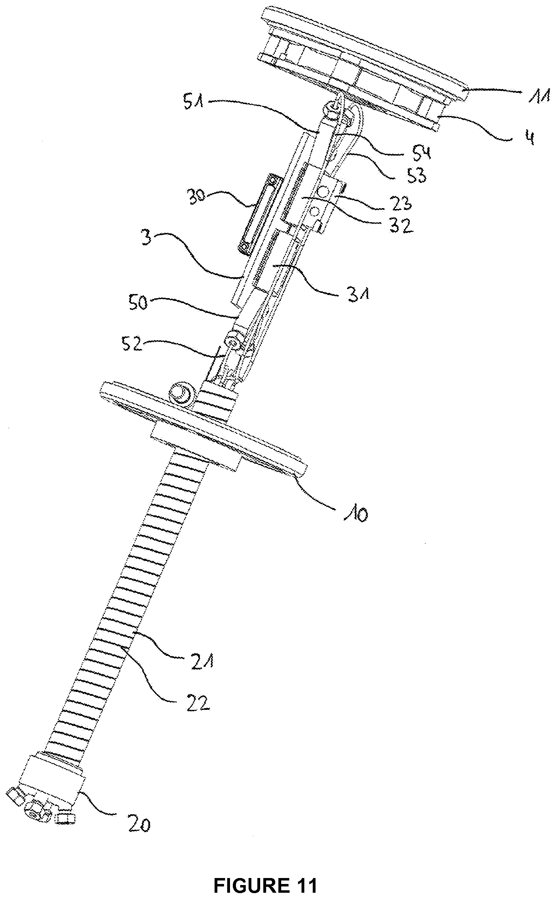

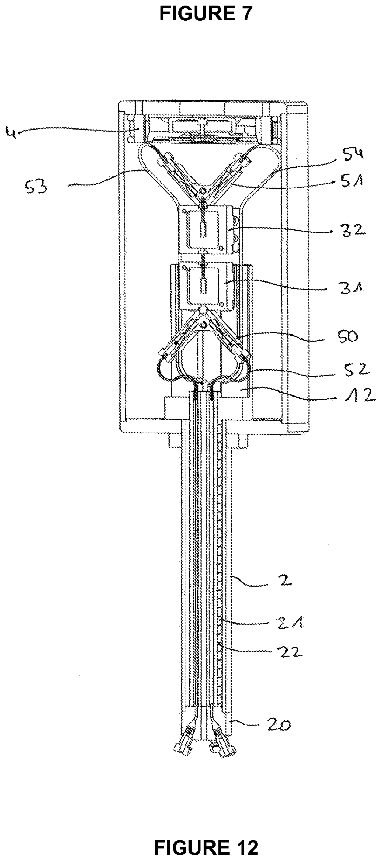

Les figures 8 à 12 illustrent un exemple de réalisation d'un détecteur comprenant une première enceinte à température régulée dans laquelle sont embarqués un détecteur NEMS et un détecteur TCD. La figure 8 est une vue en perspective du détecteur agencé dans ladite première enceinte ; les figures 9 à 12 sont des vues en coupe partielle selon différents angles.

Les détecteurs TCD et NEMS sont montés en série sur une carte de circuit imprimé 3. Les détecteurs sont agencés sous la forme de modules 31, 32 connectés électriquement au circuit imprimé 3. Par exemple, le module 31 comprend un ou plusieurs détecteurs NEMS et le module 32 comprend un ou plusieurs détecteurs TCD. Chaque module peut comprendre deux détecteurs du même type, l'un servant de référence et l'autre étant utilisé pour l'analyse en vue de la mesure différentielle mentionnée plus haut.

Selon un autre mode de réalisation (non illustré), un premier module comprend un réseau de résonateurs NEMS avec une couche fonctionnelle polaire et un second module comprend un réseau de résonateurs avec une couche fonctionnelle différente, par exemple apolaire. Toute autre configuration des modules est naturellement envisageable.

De manière particulièrement avantageuse, chaque module forme une seconde enceinte à température régulée dans laquelle est encapsulé le résonateur ou réseau de résonateurs.

La première enceinte est formée par assemblage d'une coque cylindrique 1 et de deux flasques 10, 11 agencés aux extrémités de la coque. La coque 1 présente une ouverture 13 pour le passage d'un connecteur 30 monté sur le circuit imprimé 3 et servant à connecter électriquement les détecteurs à un système de traitement extérieur. Les flasques 10, 11 présentent des ouvertures (évents) 100, 110 permettant une évacuation plus rapide de la chaleur. Naturellement, on pourrait choisir toute autre structure pour l'enceinte sans pour autant sortir du cadre de la présente invention.

Un ventilateur 4 est agencé à une extrémité de l'enceinte, le plan de rotation des pales étant perpendiculaire à l'axe longitudinal de la coque 1.

A l'extrémité opposée au ventilateur est agencé un mandrin 21 autour duquel est agencé l'élément chauffant 22 qui se présente sous la forme d'un filament chauffant enroulé autour du mandrin. Le mandrin 21 et l'élément chauffant 22 sont agencés dans un tube 2.

A l'intérieur du mandrin 21 passent des conduits capillaires adaptés pour être connectés fluidiquement à la colonne de chromatographie via un connecteur 20. Ce connecteur comprend deux entrées 201, 202 et deux sorties 203, 204. Les deux entrées alimentent les deux détecteurs (mesure et référence). Les deux sorties issues des deux détecteurs permettent de connecter en série et en aval de ceux-ci d'autres détecteurs ou un évent. Ainsi, les conduits capillaires ne présentent pas de point froid susceptible de causer une condensation de l'échantillon.

A l'intérieur de la première enceinte est agencé un support 12 destiné à supporter le circuit imprimé 3 ainsi que les conduits capillaires 52, 53, 54 qui assurent une liaison fluidique entre la colonne et les détecteurs. Le conduit 52 permet d'introduire l'échantillon dans le module 31 via le dispositif d'entrée 51 ; les conduits 53 et 54 sont agencés symétriquement de part et d'autre des modules 31, 32. Ils permettent de transférer l'échantillon du détecteur NEMS vers le connecteur 20.

Le support 12 supporte également un bloc chauffant 23 comprenant une cartouche chauffante et une sonde de température permettant de suivre en temps réel la température dans la première enceinte.

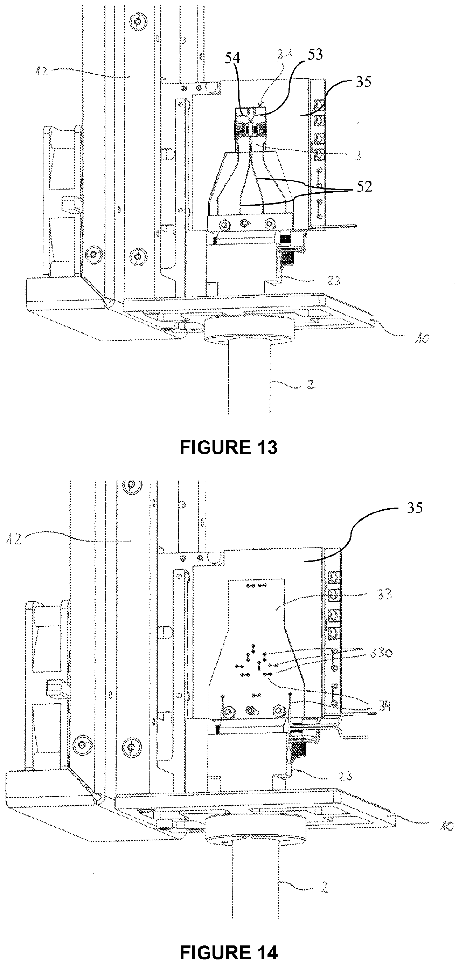

Les figures 13 et 14 présentent deux vues en coupe partielle d'un module 31 contenant un détecteur NEMS. L'agencement des composants dans la première enceinte est légèrement différent de celui des figures 8-12, mais le module des figures 13-14 peut être utilisé dans ce mode de réalisation moyennant quelques adaptations à la portée de l'homme du métier. Par rapport au mode de réalisation des figures 8-12, le bloc chauffant 23 est agencé dans la première enceinte sur le flasque 10, en regard du tube 2 contenant le mandrin et le fil chauffant enroulé sur celui-ci. Les conduits capillaires agencés dans le mandrin passent au travers du bloc 23 dans lequel ils sont chauffés à une température adéquate, afin de déboucher dans le module proprement dit.

Le module 31 comprend un empilement de plaques électriquement et thermiquement isolantes, par exemple en mica, qui forment une seconde enceinte autour du résonateur NEMS et des conduits capillaires qui sont en liaison fluidique avec celui-ci. Le résonateur est agencé sur une puce connectée électriquement au circuit imprimé 3, au centre du rectangle désigné par la référence 31. Seule une partie dudit circuit imprimé est visible sur la figure 13, le reste étant masqué par une plaque isolante 35 qui définit un volume intérieur dans lequel sont agencés le résonateur et les conduits capillaires. Ce volume est fermé par une plaque 33 visible sur la figure 14. Ladite plaque 33 est pourvue d'une pluralité de trous 330 au travers desquels un fil chauffant 34 présentant une forte résistivité électrique est passé. Les trous 330 sont agencés sur la surface de la plaque 33 de sorte que le parcours du fil chauffant permette un chauffage homogène des conduits capillaires et du résonateur NEMS. Bien que non visible sur les figures 13 et 14, un capteur de température est agencé dans le module 31 afin de mesurer en temps réel la température à l'intérieur dudit module. Ladite température est asservie à un profil de température déterminé au moyen d'une boucle d'asservissement qui comprend un calculateur (non représenté) connecté d'une part au capteur de température, dont il reçoit les données de mesure, et à une source électrique reliée au fil chauffant, à laquelle il envoie des consignes d'intensité du courant électrique à faire passer dans le fil chauffant pour atteindre la température souhaitée. De manière avantageuse, le calculateur est embarqué sur le circuit imprimé 3, de sorte que le détecteur est entièrement autonome. Le volume intérieur de la seconde enceinte étant extrêmement faible, la température peut y être contrôlée en temps réel de manière très fine sur une large gamme de température.

Naturellement, ces agencements particuliers du détecteur sont donnés uniquement à titre d'exemples non limitatifs. En particulier, l'élément chauffant peut se présenter sous une autre forme qu'un fil résistif, par exemple sous la forme d'une barre ou d'une plaque plane, ce qui permet de simplifier son assemblage sur une paroi de la seconde enceinte, notamment en vue d'une production à l'échelle industrielle. Tout autre élément chauffant adapté aux dimensions de la seconde enceinte peut être utilisé.

Afin de couvrir une large gamme de température de travail du détecteur, les matériaux qui le constituent sont choisis pour résister à une température de l'ordre de 300°C.

La coque et les flasques qui définissent l'enveloppe de la première enceinte sont typiquement réalisés en métal.

S'agissant de la carte de circuit imprimé qui supporte le résonateur, le matériau privilégié est une céramique ou du polyimide (Kapton^{™}) par exemple.

Le résonateur NEMS et, le cas échéant, le détecteur TCD, sont avantageusement réalisés en silicium dopé ou en nitrure de silicium assurant la rigidité mécanique et la conduction électrique. Le silicium dopé est en effet capable de supporter des températures de plus de 400°C sans modification de ses propriétés électroniques et mécaniques. Le détecteur TCD est enrobé d'une couche de platine conservant toutes ses propriétés physiques bien au-delà de 300°C.

En ce qui concerne la couche fonctionnelle, les polymères qui sont utilisés classiquement pour fonctionnaliser des résonateurs NEMS ne sont généralement pas utilisables dans la présente invention, car ils résistent mal à la chaleur. En effet, la température de dépôt de ces polymères est de l'ordre de 100°C à 200°C selon leur nature. Par conséquent, dans la présente invention, le résonateur NEMS est avantageusement fonctionnalisé avec une couche d'un oxyde poreux issu de la microélectronique déposé à haute température (de l'ordre de 400 à 500°C) qui offre de bonnes réponses chimiques sur une large gamme de molécules (alcane, alcène, alcool, composés aromatiques, etc.). De manière avantageuse, la composition dudit oxyde est de formule générale SiOₓC_{y}H_{z} (avec x > 0 et y et z ≥ 0), par exemple SiOC, SiO₂, SiOH... Un tel oxyde poreux est notamment décrit dans le document WO/2015/097282. Un oxyde d'alumine, de formule générale AlₓO_{y} (avec x et y > 0), par exemple Al₂O₃, pourrait également être utilisé.

Pour réaliser la veine fluidique englobant le résonateur NEMS et le détecteur TCD, on assemble au substrat 1000 qui les supporte un capot structuré 2000 en verre ou silicium avec du verre fritté 2001 (« glass frit » selon la terminologie anglo-saxonne) dont le procédé de thermosoudure est réalisé aux alentours de 400°C. La figure 15 est une vue en coupe de ladite veine fluidique.

Les capillaires 3000, 3001 pour amener les gaz sur la puce sont en verre et collés au moyen d'une colle époxy réticulée à haute température, par exemple commercialisée sous le nom de EPO-TEK^{®} 731, désignée par le repère 2002.

Le respect de ces précautions dans le choix des matériaux permet d'assurer une bonne tenue du détecteur à des températures de travail élevées.

Par ailleurs, compte tenu de l'encombrement réduit du détecteur et de la colonne de chromatographie, il est possible de miniaturiser le système d'analyse de gaz, afin par exemple de l'embarquer dans un dispositif transportable dont le diamètre est d'une dizaine de centimètres ou davantage. Par ailleurs, le faible débit de fluide vecteur nécessaire pour l'entraînement de l'échantillon et la faible consommation énergétique pour la régulation de température de la colonne de chromatographie et du détecteur NEMS permettent une grande autonomie du système. Ainsi, le système peut être utilisé pour effectuer des analyses dans des endroits peu accessibles, y compris en environnement dangereux.

Par exemple, le système d'analyse de gaz peut être inséré dans un véhicule sousmarin autonome et programmable (par exemple dans une torpille) ou dans un outil de forage, et permettre ainsi de mettre en œuvre les analyses décrites plus haut in situ dans les océans et les réservoirs.

L'intégration d'une instrumentation au sein de torpilles et d'outils de forage est connue pour d'autres types de capteurs, et ne sera donc pas décrite plus en détail dans le présent texte.

### REFERENCES

[Mile2010] E. Mile, G. Jourdan, I. Bargatin, S. Labarthe, C. Marcoux, P. Andreucci, S. Hentz, C. Kharrat, E. Colinet, L. Duraffourg, In-plane nanoelectromechanical resonators based on silicon nanowire piezoresistive détection, Nanotechnology 21, (2010) 165504
[Bao2007] M. Bao, H. Yang, Squeeze film air damping in MEMS, Sensors and Actuators A 136 (2007) 3-27
EP 2 008 965
WO 2012/034990
WO 2012/034951
WO 2014/053575
EP 2 878 942
WO/2015/097282

## Revendications

1. Procédé d'analyse d'hydrocarbure, comprenant :
- la mise en œuvre d'une séparation chromatographique en phase gazeuse selon un premier profil de température contrôlé, pour séparer un échantillon en une pluralité d'analytes ;
- la détection d'au moins un desdits analytes par mesure d'une variation de la fréquence de résonance d'au moins un résonateur de type nano-système électromécanique (NEMS) recouvert d'une couche fonctionnelle entraîné en vibration à sa fréquence de résonance, sous l'effet d'une adsorption ou désorption de l'analyte par la couche fonctionnelle,
ledit procédé étant **caractérisé en ce que** le résonateur est soumis à un second profil de température contrôlé, inférieur au premier profil de sorte qu'à chaque instant, la température appliquée au résonateur est inférieure à la température appliquée à la colonne de chromatographie au sein de laquelle la séparation est effectuée, chaque profil de température étant appliqué par des enceintes à température régulée respectives découplées thermiquement l'une de l'autre.

2. Procédé selon la revendication 1, dans lequel l'écart de température entre le premier profil et le second profil est compris entre 5 et 150°C, de préférence entre 30 et 100°C, de manière encore préférée entre 40 et 60°C.

3. Procédé selon l'une des revendications 1 ou 2, dans lequel la température du premier profil évolue entre 50 et 400°C.

4. Procédé selon l'une des revendications 1 à 3, dans lequel la température du second profil évolue entre 0 et 350°C.

5. Procédé selon l'une des revendications 1 à 4, dans lequel la détection d'au moins un desdits analytes comprend en outre la mesure d'une variation de l'amplitude de résonance du résonateur.

6. Procédé selon l'une des revendications 1 à 5, dans lequel l'échantillon comprend des chaînes carbonées présentant entre 16 et 40 atomes de carbone.

7. Procédé selon l'une des revendications 1 à 6, dans lequel le résonateur est encapsulé dans une enceinte à température régulée, ladite enceinte comprenant une unité de régulation de la température configurée pour faire varier la température à l'intérieur de l'enceinte de manière contrôlée.

8. Procédé selon la revendication 7, dans lequel la colonne de chromatographie est encapsulée dans une enceinte découplée thermiquement de l'enceinte dans laquelle est encapsulé le résonateur.

9. Procédé selon l'une des revendications 1 à 8, comprenant en outre une analyse dudit au moins un analyte par un catharomètre agencé dans un même conduit fluidique que le résonateur.

10. Procédé selon l'une des revendications 1 à 9, comprenant la mise en œuvre d'un traitement pour soustraire de la ligne de base de la réponse du résonateur la ligne de base d'une réponse du résonateur, dite réponse à blanc, préalablement mesurée en l'absence de circulation d'un fluide pour un même profil de température.

11. Procédé selon l'une des revendications 1 à 9, comprenant en outre la mise en œuvre d'une mesure de la variation de la fréquence de résonance d'au moins un second résonateur, dit résonateur de référence, soumis au même profil de température mais non exposé à l'analyte, et la mise en œuvre d'un traitement configuré pour soustraire du signal de réponse du résonateur exposé à l'analyte le signal de réponse du résonateur de référence.

12. Procédé selon l'une des revendications 1 à 11, dans lequel on déduit de la mesure de la variation de la fréquence de résonance du résonateur la composition moléculaire de l'analyte.

13. Procédé selon la revendication 12 en combinaison avec la revendication 5, dans lequel on déduit en outre de la mesure de la variation de l'amplitude de résonance du résonateur une caractéristique fluidique de l'analyte.

14. Procédé de comparaison de deux coupes pétrolières, dans lequel on met en œuvre le procédé selon l'une des revendications 1 à 13 pour analyser la composition de chacune desdites coupes.

15. Procédé pour doser un composé déterminé dans un hydrocarbure, dans lequel on met en œuvre le procédé selon l'une des revendications 1 à 13 pour détecter ledit composé au sein d'un échantillon d'hydrocarbure.

16. Procédé pour doser un hydrocarbure déterminé dans de l'eau, dans lequel on met en œuvre le procédé selon l'une des revendications 1 à 13 pour détecter ledit hydrocarbure au sein d'un échantillon d'eau.

17. Outil de forage ou véhicule autonome pour l'exploitation d'hydrocarbures et/ou l'exploration sous-marine, comprenant un système d'analyse pour la mise en œuvre du procédé selon l'une des revendications précédentes, ledit système comprenant :
- une colonne de chromatographie en phase gazeuse, agencée dans une première enceinte à température contrôlée,
- un résonateur de type nano-système électromécanique (NEMS) agencé dans un conduit fluidique en sortie de la colonne de chromatographie, ledit résonateur comprenant une couche fonctionnelle et étant agencé dans une seconde enceinte à température contrôlée,
- un dispositif de lecture adapté pour entraîner le résonateur en vibration à sa fréquence de résonance et pour mesurer une variation de ladite fréquence de résonance sous l'effet de l'adsorption ou de la désorption de l'analyte par la couche fonctionnelle,
la première et la seconde enceinte étant découplées thermiquement l'une de l'autre, chaque enceinte comprenant une unité de régulation de la température, lesdites unités de régulation étant configurées pour faire varier la température dans leur enceinte respective selon des profils différents, de sorte qu'à chaque instant la température appliquée au résonateur soit inférieure à la température appliquée à la colonne de chromatographie.

18. Outil ou véhicule selon la revendication 17, dans lequel le système d'analyse comprend en outre un catharomètre agencé dans le conduit fluidique, en amont ou en aval du résonateur.

## Patentansprüche

1. Kohlenwasserstoff-Analyseverfahren, umfassend:
- das Umsetzen einer chromatographischen Trennung in der Gasphase gemäß einem ersten kontrollierten Temperaturprofil zum Trennen einer Probe in eine Vielzahl von Analyten,
- die Detektion wenigstens eines der genannten Analyten per Messung einer Variation der Resonanzfrequenz wenigstens eines Resonators von Typ elektromechanisches Nanosystem (NEMS), das mit einer funktionalen Schicht abgedeckt wird, die in Vibration in ihrer Resonanzfrequenz unter der Wirkung einer Adsorption oder Desorption des Analyts durch die funktionale Schicht angetrieben wird;
wobei das genannte Verfahren **dadurch gekennzeichnet ist, dass** es einem zweiten kontrollierten Temperaturprofil unterworfen wird, das derart niedriger ist als das erste Profil, dass die auf den Resonator angewendete Temperatur jederzeit niedriger ist als die auf die Chromatographiesäule angewendete Temperatur, in der die Trennung erfolgt, wobei jedes Temperaturprofil durch jeweilige Einfassungen mit regulierter Temperatur, die thermisch voneinander entkoppelt werden, angewendet wird.

2. Verfahren gemäß Anspruch 1, bei dem der Temperaturunterschied zwischen dem ersten Profil und dem zweiten Profil zwischen 5 und 150 °C, bevorzugt zwischen 30 und 100 °C, weiter bevorzugt zwischen 40 und 60 °C inbegriffen ist.

3. Verfahren gemäß einem der Ansprüche 1 oder 2, bei dem die Temperatur des ersten Profils zwischen 50 und 400 °C schwankt.

4. Verfahren gemäß einem der Ansprüche 1 bis 3, bei dem die Temperatur des zweiten Profils zwischen 0 und 350 °C schwankt.

5. Verfahren gemäß einem der Ansprüche 1 bis 4, bei dem die Detektion wenigstens eines der genannten Analyten darüber hinaus die Messung einer Variation der Resonanzamplitude des Resonators umfasst.

6. Verfahren gemäß einem der Ansprüche 1 bis 5, bei dem die Probe kohlenstoffhaltige Ketten umfasst, die zwischen 16 und 40 Kohlenstoffatome aufweisen.

7. Verfahren gemäß einem der Ansprüche 1 bis 6, bei dem der Resonator in einer Einfassung mit regulierter Temperatur verkapselt ist, wobei die genannte Einfassung eine Regulierungseinheit der Temperatur ist, die ausgestaltet ist, um die Temperatur im Innern der Einfassung auf kontrollierte Weise zum Variieren zu bringen.

8. Verfahren gemäß Anspruch 7, bei dem die Chromatographiesäule in einer Einfassung verkapselt ist, die thermisch von der Einfassung entkoppelt ist, in der der Resonator verkapselt ist.

9. Verfahren gemäß einem der Ansprüche 1 bis 8, umfassend darüber hinaus eine Analyse des genannten wenigstens einen Analyten durch ein Katharometer, das in einer und derselben fluidischen Leitung angeordnet ist wie der Resonator.

10. Verfahren gemäß einem der Ansprüche 1 bis 9, umfassend das Umsetzen einer Verarbeitung, um der Grundlinie der Antwort des Resonators die Grundlage einer Antwort des Resonators, bezeichnet als "leere Antwort" zu entziehen, die zuvor bei Fehlen des Umlaufs einer Flüssigkeit für ein und dasselbe Temperaturprofil gemessen wurde.

11. Verfahren gemäß einem der Ansprüche 1 bis 9, umfassend darüber hinaus das Umsetzen einer Messung der Variation der Resonanzfrequenz wenigstens eines zweiten Resonators, bezeichnet als Referenzresonator, der demselben Temperaturprofil unterworfen ist, jedoch nicht dem Analyten ausgesetzt wird, und das Umsetzen einer Verarbeitung, die ausgestaltet ist, um dem Antwortsignal des Resonators, der dem Analyten ausgesetzt ist, das Antwortsignal des Referenzresonators zu entziehen.

12. Verfahren gemäß einem der Ansprüche 1 bis 11, bei dem von der Messung der Variation der Resonanzfrequenz des Resonators die Molekülzusammensetzung des Analyts abgeleitet wird.

13. Verfahren gemäß Anspruch 12 in Verbindung mit Anspruch 15, bei dem darüber hinaus die Messung der Variation der Resonanzamplitude des Resonators ein fluidisches Merkmal des Analyts abgeleitet wird.

14. Vergleichsverfahren von zwei Erdölschnitten, bei dem das Verfahren gemäß einem der Ansprüche 1 bis 13 zum Analysieren der Zusammensetzung von jedem der genannten Schnitte umgesetzt wird.

15. Verfahren zum Dosieren einer bestimmten Verbindung in einem Kohlenwasserstoff, bei dem das Verfahren gemäß einem der Ansprüche 1 bis 13 zum Detektieren der genannten Verbindung innerhalb einer Kohlenwasserstoff-Probe umgesetzt wird.

16. Verfahren zum Dosieren eines bestimmten Kohlenwasserstoffes in Wasser, bei dem das Verfahren gemäß einem der Ansprüche 1 bis 13 umgesetzt wird, um den genannten Kohlenwasserstoff innerhalb einer Wasserprobe zu detektieren.

17. Bohrwerkzeug oder autonomes Fahrzeug zur Ausbeutung von Kohlenwasserstoffen und/oder Unterwasser-Exploration, umfassend ein Analysesystem für die Umsetzung des Verfahrens gemäß einem der voranstehenden Ansprüche, wobei das genannte System umfasst:
- eine Chromatographiesäule in der Gasphase, die in einer ersten Einfassung mit kontrollierter Temperatur angeordnet ist,
- einen Resonator vom Typ elektromechanisches Nanosystem (NEMS), das in einer fluidischen Leitung am Ausgang der Chromatographiesäule angeordnet ist, wobei der genannte Resonator eine funktionale Schicht umfasst und in einer zweiten Einfassung mit kontrollierter Temperatur angeordnet ist,
- eine Lesevorrichtung, die zum Antreiben des Resonators in Vibration bei ihrer Resonanzfrequenz und zum Messen einer Variation der genannten Resonanzfrequenz unter der Wirkung der Adsorption oder der Desorption des Analyts durch die funktionale Schicht geeignet ist,
wobei die erste und die zweite Einfassung thermisch voneinander entkoppelt sind, wobei jede Einfassung eine Regulierungseinheit der Temperatur umfasst, wobei die genannten Regulierungseinheiten ausgestaltet sind, um die Temperatur in ihrer jeweiligen Einfassung gemäß unterschiedlichen Profilen derart zum Variieren zu bringen, dass die auf den Resonator angewendete Temperatur jederzeit unter der auf die Chromatographiesäule angewendeten Temperatur liegt.

18. Werkzeug oder Fahrzeug gemäß Anspruch 17, bei dem das Analysewerkzeug darüber hinaus ein Katharometer umfasst, das in der fluidischen Leitung dem Resonator vorgeschaltet oder nachgeschaltet angeordnet ist.

## Claims

1. Method for analysing hydrocarbons, comprising:
- the implementation of a gas chromatography separation according to a first controlled temperature profile, to separate a sample into a plurality of analytes;
- the detection of at least one of said analytes by measurement of a variation of the resonance frequency of at least one resonator of nano-electromechanical system (NEMS) type covered with a functional layer made to vibrate at the resonance frequency thereof, under the effect of an adsorption or desorption of the analyte by the functional layer,
said method being **characterised in that** the resonator is subjected to a second controlled temperature profile, lower than the first profile so that at any time, the temperature applied to the resonator is lower than the temperature applied to the chromatography column within which separation is performed, each temperature profile being applied by respective temperature regulated chambers thermally decoupled from each other.

2. Method according to claim 1, wherein the temperature difference between the first profile and the second profile is comprised between 5 and 150°C, preferably between 30 and 100°C, in an even more preferred manner between 40 and 60°C.

3. Method according to one of claims 1 or 2, wherein the temperature of the first profile evolves between 50 and 400°C.

4. Method according to one of claims 1 to 3, wherein the temperature of the second profile evolves between 0 and 350°C.

5. Method according to one of claims 1 to 4, wherein the detection of at least one of said analytes further comprises the measurement of a variation of the resonance amplitude of the resonator.

6. Method according to one of claims 1 to 5, wherein the sample comprises carbon chains having between 16 and 40 carbon atoms.

7. Method according to one of claims 1 to 6, wherein the resonator is encapsulated in a temperature regulated chamber, said chamber comprising a temperature regulating unit configured to make the temperature vary inside the chamber in a controlled manner.

8. Method according to claim 7, wherein the chromatography column is encapsulated in a chamber thermally decoupled from the chamber in which the resonator is encapsulated.

9. Method according to one of claims 1 to 8, further comprising an analysis of said at least one analyte by a catharometer arranged in a same fluidic conduit as the resonator.

10. Method according to one of claims 1 to 9, comprising the implementation of a processing to subtract from the base line of the response of the resonator the base line of a response of the resonator, called blank response, measured beforehand in the absence of circulation of fluid for a same temperature profile.

11. Method according to one of claims 1 to 9, further comprising the implementation of a measurement of the variation of the resonance frequency of at least one second resonator, called reference resonator, subjected to the same temperature profile but not exposed to the analyte, and the implementation of a processing configured to subtract from the response signal of the resonator exposed to the analyte the response signal of the reference resonator.

12. Method according to one of claims 1 to 11, wherein the molecular composition of the analyte is deduced from the measurement of the variation of the resonance frequency of the resonator.

13. Method according to claim 12 in combination with claim 5, wherein a fluidic characteristic of the analyte is further deduced from the measurement of the variation of the resonance amplitude of the resonator.

14. Method for comparing two petroleum fractions, wherein the method according to one of claims 1 to 13 is implemented to analyse the composition of each of said fractions.

15. Method for assaying a determined compound in a hydrocarbon, wherein the method according to one of claims 1 to 13 is implemented to detect said compound within a sample of hydrocarbon.

16. Method for assaying a determined hydrocarbon in water, wherein the method according to one of claims 1 to 13 is implemented to detect said hydrocarbon within a water sample.

17. Drilling tool or autonomous vehicle for the exploitation of hydrocarbons and/or underwater exploration, comprising an analysis system for the implementation of the method according to one of the preceding claims, said system comprising:
- a gas chromatography column, arranged in a first temperature controlled chamber,
- a resonator of nano-electromechanical system (NEMS) type arranged in a fluidic conduit at the outlet of the chromatography column, said resonator comprising a functional layer and being arranged in a second temperature controlled chamber,
- a reading device suited to make the resonator vibrate at the resonance frequency thereof and to measure a variation of said resonance frequency under the effect of the adsorption or the desorption of the analyte by the functional layer,
the first and the second chambers being thermally decoupled from each other, each chamber comprising a temperature regulating unit, said regulating units being configured to vary the temperature in their respective chamber according to different profiles, so that at any time the temperature applied to the resonator is lower than the temperature applied to the chromatography column.

18. Tool or vehicle according to claim 17, wherein the analysis system further comprises a catharometer arranged in the fluidic conduit, upstream or downstream of the resonator.
